# EUROPÄISCHE PATENTANMELDUNG

(11) **EP 2 695 636 A1**
(43) Veröffentlichungstag der Anmeldung: **12.02.2014**
(21) Anmeldenummer: 13174056.5
(22) Anmeldetag: 27.06.2013
(51) Int. Cl.: A61M 25/00

(54) **Katheter-Set**

(30) Priorität: 10.08.2012 DE 102012214259
(71) Anmelder: Manfred Sauer GmbH, 74931 Lobbach (DE)
(72) Erfinder: Sauer, Manfred, 74931 Lobbach (DE); Katzenberger, Uwe, 74934 Reichartshausen (DE)
(74) Vertreter: Ullrich & Naumann

(57) **Zusammenfassung**

Ein Katheter-Set umfassend einen zur Selbstkatheterisierung geeigneten Katheter (2) mit einer gelbeschichteten Oberfläche (3), wobei sich der Katheter (2) gebrauchsfertig in einer flüssigkeitsdichten, schlauchartigen Gebrauchsverpackung (1) befindet, ist dadurch gekennzeichnet, dass die Gebrauchsverpackung (1) gasdurchlässig ausgebildet ist und dass mindestens zwei Gebrauchsverpackungen (1) mit jeweils darin befindlichem Katheter (2) gemeinsam in einer gasundurchlässigen Lagerverpackung (7) untergebracht sind.

## Beschreibung

Die Erfindung betrifft ein Katheter-Set umfassend einen zur Selbstkatheterisierung geeigneten Katheter mit einer gelbeschichteten Oberfläche, wobei sich der Katheter gebrauchsfertig in einer flüssigkeitsdichten, schlauchartigen Gebrauchsverpackung befindet.

Gattungsbildende Katheter bzw. Katheter-Sets sind in den unterschiedlichsten Ausprägungen aus der Praxis bekannt. Die hier in Rede stehenden Katheter dienen zur Selbstkatheterisierung und werden regelmäßig von Menschen mit eingeschränkter oder nicht mehr vorhandener Blasenfunktion verwendet, so beispielsweise von Querschnittsgelähmten.

Zum einschlägigen druckschriftlichen Stand der Technik sei beispielsweise auf die WO 98/19729 verwiesen. Aus dieser Druckschrift ist ein gebrauchsfertiger Katheter bekannt, bei dem der Katheterschaft mit einer hydrophilen Oberfläche bzw. Beschichtung versehen ist. In Verbindung mit einer Benetzungsflüssigkeit quillt die hydrophile Oberfläche leicht auf und ruft eine gleitende Eigenschaft hervor, so dass zum Einführen des Katheters keine weiteren Gleitmittel erforderlich sind.

Die bekannten Katheter mit hydrophiler Oberfläche/Beschichtung werden entweder im trockenen Zustand bereitgestellt und müssen zur Herbeiführung des gebrauchsfertigen Zustands gewässert bzw. mit Benetzungsflüssigkeit in Kontakt gebracht werden. Dies kann dadurch geschehen, dass in die Gebrauchsverpackung Benetzungsflüssigkeit hineingegeben oder aus einem innerhalb der Gebrauchsverpackung angeordneten Behältnis Benetzungsflüssigkeit freigesetzt wird. In beiden Fällen ist eine Einwirkzeit zur Aktivierung der hydrophilen Oberfläche erforderlich.

Gebrauchsfertige Katheter werden auch bereits insoweit angeboten, als diese innerhalb der Gebrauchsverpackung im gewässerten Zustand bereitgestellt werden. Der Katheter befindet sich somit in einem Bad von Benetzungsflüssigkeit. Dies erfordert eine ganz besondere Gebrauchsverpackung, nämlich eine flüssigkeits- und gasdichte Gebrauchsverpackung, um nämlich zu vermeiden, dass Benetzungsflüssigkeit verdampft und der Innenraum der Gebrauchsverpackung - mit dem Katheter - austrocknet. Ist die Gebrauchsverpackung nicht gasundurchlässig ausgeführt, reduziert sich die Haltbarkeit bzw. Lagerfähigkeit des Katheter-Sets ganz erheblich.

Die Vorkehrung einer gasundurchlässigen Gebrauchsverpackung erfordert jedoch ganz besondere konstruktive Maßnahmen, nämlich in Bezug auf das Material der Gebrauchsverpackung. Eine nahezu hundertprozentige Gasdichtigkeit lässt sich nur durch eine entsprechend ausgelegte Barriere erzielen, nämlich beispielsweise durch Gebrauchsverpackungen aus Kunststoff mit einer Aluminiumkaschierung. Solche Gebrauchsverpackungen haben jedoch den großen Nachteil, dass sie in sich äußerst steif sind. Folglich lässt sich eine solche Gebrauchsverpackung ausschließlich zu reinen Verpackungszwecken verwenden und nur mühsam handhaben. Aufgrund des komplexen Aufbaus sind die Herstellungskosten - einer jeden Gebrauchsverpackung - erheblich.

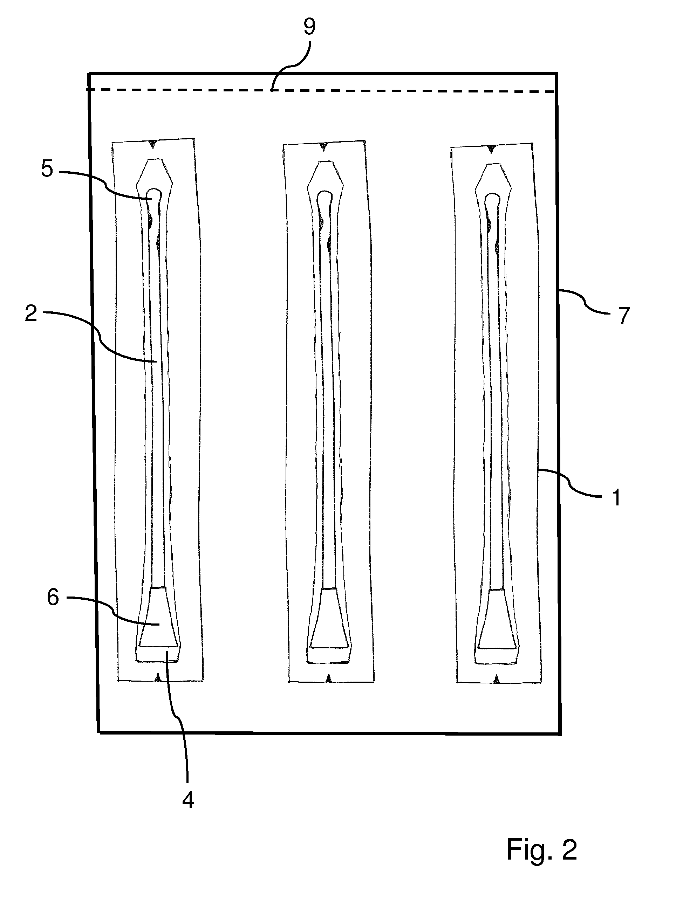

Aus der EP 1 175 355 B1 ist ein Katheter mit hydrophiler Oberfläche bekannt, entsprechend den voranstehenden Ausführungen. Im Rahmen eines dort in Figur 2 gezeigten weiteren Ausführungsbeispiels ist der Katheter nicht nur in einer Gebrauchsverpackung untergebracht, sind vielmehr zwei oder mehrere Katheter, die sich jeweils in einer Gebrauchsverpackung befinden, gemeinsam in einer Lagerverpackung untergebracht. Durch diese Maßnahme soll die Lagerbarkeit der bereits gewässerten hydrophilen Katheter begünstigt werden.

Der vorliegenden Erfindung liegt nun die Aufgabe zugrunde, ein Katheter-Set der gattungsbildenden Art derart auszugestalten und weiterzubilden, dass die Gebrauchsverpackung wenig aufwendig in der Herstellung, einfach zu handhaben und obendrein zu weiteren Zwecken, abseits der eigentlichen Bereitstellung, nutzbar ist. Außerdem soll der Katheter eine marktfähige Alternative zu den Kathetern mit hydrophiler Oberfläche darstellen, insbesondere die dort aufwändige Herstellung zur Erzeugung der hydrophilen Beschichtung vereinfachen.

Die voranstehende Aufgabe ist durch ein Katheter-Set mit den Merkmalen des Patentanspruchs 1 gelöst. Danach ist das gattungsbildende Katheter-Set dadurch gekennzeichnet, dass die Gebrauchsverpackung gasdurchlässig ausgebildet ist und dass mindestens zwei Gebrauchsverpackungen mit jeweils darin befindlichem Katheter gemeinsam in einer gasundurchlässigen Lagerverpackung untergebracht sind.

Erfindungsgemäß ist erkannt worden, dass es durchaus möglich ist, einzeln verpackte Katheter im gebrauchsfertigen Zustand bereitzustellen, und zwar ohne dass die Gebrauchsverpackung eines jeden Katheters gasimpermeabel, d. h. gasundurchlässig, ausgeführt ist. Zum Vorhalten eines gebrauchsfertigen gelbeschichten Katheters, d. h. eines Katheters mit relativ dünnflüssiger Gelbeschichtung bzw. eines gewässerten Gel-Katheters, ist es erforderlich, dass die Gebrauchsverpackung flüssigkeitsdicht, jedoch nicht gasundurchlässig, ausgebildet ist. Des Weiteren ist erkannt worden, dass man zwei oder mehrere der jeweils in einer Gebrauchsverpackung befindlichen Katheter zusammenfassen kann, nämlich in einer Lagerverpackung. Mit anderen Worten wird die Gasdurchlässigkeit einer jeden Gebrauchsverpackung dadurch kompensiert, dass mehrere Gebrauchsverpackungen - gemeinsam - mit darin befindlichen Kathetern und deren Gelbeschichtung in einer gasundurchlässigen Lagerverpackung untergebracht sind. Die so verpackten Katheter lassen sich über einen längeren Zeitraum lagern, nämlich aufgrund der gasundurchlässigen Lagerverpackung. Die einzelnen Gebrauchsverpackungen, d.h. die für jeden einzelnen Katheter erforderlichen Verpackungen, lassen sich einfachst generieren, nämlich durch ein flüssigkeitsdichtes, jedoch gasdurchlässiges Material. Entsprechend ist es möglich, das Material der schlauchartigen Gebrauchsverpackung so dünn und flexibel wie möglich auszugestalten, um nämlich nach dem Öffnen der Gebrauchsverpackung den Katheter auf einfache Weise, ohne diesen zu berühren, aus der Gebrauchsverpackung auszubringen, nämlich dadurch, dass die Gebrauchsverpackung entlang dem Katheterschaft ziehharmonikaartig zurückgestülpt bzw. zurückgerafft wird, so dass zunächst die Katheterspitze und nach und nach der gesamte Katheterschaft freigelegt wird.

In vorteilhafter Weise befindet sich der Katheter in einem sterilen Zustand in der Gebrauchsverpackung. Dies bedeutet, dass der Katheter sich tatsächlich im gebrauchsfertigen Zustand in der Gebrauchsverpackung befindet, ohne einen Sterilisierungsvorgang bzw. eine Entkeimung vornehmen zu müssen. Im Konkreten ist es denkbar, dass der Katheter vor oder nach seiner Anordnung in der Gebrauchsverpackung, nach einer herkömmlichen Methode, sterilisiert worden ist. Es ist grundsätzlich möglich, den Katheter durch Gaseinwirkung, durch Temperatureinwirkung oder durch Bestrahlung zu sterilisieren.

Ebenso ist es denkbar, dass der Katheter vor oder nach der Kontaktierung mit Benetzungsflüssigkeit, ebenfalls nach einer herkömmlichen Methode entsprechend den voranstehenden Ausführungen, sterilisiert worden ist. Jede nur denkbare Methode zur Sterilisierung des Katheters ist anwendbar.

In Bezug auf die Gebrauchsverpackung ist es von ganz besonderem Vorteil, wenn diese im Bereich der Katheterspitze öffenbar bzw. aufreißbar ist. Dazu kann eine Art Sollbruchstelle, Einkerbung, ein Einschnitt oder dergleichen in der Gebrauchsverpackung vorgesehen sein. Zum Gebrauch des Katheters wird jedenfalls die Gebrauchsverpackung im vorderen Bereich geöffnet und lässt sich der Katheter durch Zusammenraffen des Materials der Katheterverpackung allmählich aus der Gebrauchsverpackung herausführen bzw. lässt sich die Gebrauchsverpackung zu dem der Katheterspitze abgewandten Ende hin zusammenfalten, zusammenraffen bzw. zusammenschieben, wodurch der Katheter von der Gebrauchsverpackung nach und nach befreit wird.

Auch ist es denkbar, dass die Gebrauchsverpackung in einem der Katheterspitze abgewandten Bereich öffenbar bzw. aufreißbar ist, um nämlich einen rückwärtigen Auslauf zu bilden.

In weiter vorteilhafter Weise ist die Gebrauchsverpackung derart ausgestaltet, dass der Katheter mit der Katheterspitze voran zumindest teilweise aus der geöffneten bzw. aufgerissenen Gebrauchsverpackung herausschiebar oder herausziehbar ist. Sofern der Katheter mit einem endseitigen Anschlussstück ausgestattet ist, lässt sich dieser im Wesentlichen bis zum Anschlussstück aus der Gebrauchsverpackung herausziehen und mit dem Anschlussstück dort festklemmen. Dabei könnte sich das Anschlussstück zum freien Ende hin konisch erweitern. Die Gebrauchsverpackung weist im öffenbaren Bereich eine entsprechend angepasste Innenwandung auf, so dass sich das Anschlussstück beim Herausschieben des Katheters in diesen Bereich schieben und dort abdichtend festklemmen lässt. Mit anderen Worten kommt das Anschlussstück im herausgezogenen Zustand des Katheters zur zumindest weitgehend abdichtenden Anlage an bzw. in der Gebrauchsverpackung, nämlich dergestalt, dass die Gebrauchsverpackung als schlauchartige Verlängerung des Katheters zur unmittelbaren Urinableitung, beispielweise in eine Toilette, dient. Somit kommt der Gebrauchsverpackung eine doppelte Funktion zu, nämlich einerseits die Funktion der sterilen Bereitstellung bei flüssigkeitsdichter Ausgestaltung und andererseits die Funktion zur weiterreichenden Urinableitung, nämlich aus der Blase heraus durch den Katheter und schließlich durch die eine Verlängerung des Katheters bildende Gebrauchsverpackung hindurch in eine Toilette.

In Bezug auf die konkrete Ausgestaltung und das Material der Gebrauchsverpackung ist es von Vorteil, wenn diese aus einer laminierten Folie, vorzugsweise zweilagig, hergestellt ist. Die Außenschicht der Folie könnte aus Polyamid (PA) und die Innenschicht aus Polyethylen (PE) bestehen.

Des Weiteren ist es von Vorteil, wenn die Folie dünn und flexibel ausgeführt ist, so dass sie sich zur Freigabe des Katheters mühelos zusammenschieben bzw. zusammenraffen lässt. Im Rahmen einer solchen Ausgestaltung ist die weitere Funktion der schlauchartigen Gebrauchsverpackung begünstigt, nämlich dahingehend, dass sich diese mühelos als Verlängerung des Katheters handhaben lässt, nämlich entsprechend den voranstehenden Ausführungen.

Wie bereits zuvor ausgeführt, dient die Lagerverpackung zur längerfristigen Lagerung bzw. Bereitstellung mehrerer Gebrauchsverpackungen mit darin befindlichen Kathetern nebst Benetzungsflüssigkeit. Die Vorkehrung der Lagerverpackung ermöglicht die Langzeitlagerung der Katheter in einer gasundurchlässigen Umverpackung, wobei die Lagerverpackung in Form eines langgestreckten Briefs, Beutels oder dergleichen ausgeführt sein kann. Zum erstmaligen Öffnen an vorgegebener Stelle ist diese vorzugsweise entlang einer Kante aufreißbar. Dazu kann eine Sollbruchstelle, eine Einkerbung, Perforation oder können sonstige Maßnahmen zur Begünstigung des Öffnens vorgesehen sein. Auch ist es denkbar, dass die Lagerpackung vorzugsweise über eine Druckleiste oder dergleichen reversibel öffenbar/schließbar ist, um nämlich die Gasundurchlässigkeit der Lagerverpackung auch nach erstmaligem Öffnen weiterhin gewährleisten zu können. Letztendlich eignet sich die Lagerverpackung in ganz besonderem Maße als Transportverpackung mit mehreren darin befindlichen Gebrauchsverpackungen nebst Katheter.

Die Lagerverpackung ist in weiter vorteilhafter Weise derart dimensioniert, dass sie zur Aufnahme mehrerer Gebrauchsverpackungen mit jeweils einem Katheter in der Gebrauchsverpackung geeignet ist. So lassen sich in der Lagerverpackung beispielsweise 5 bis 20 Gebrauchsverpackungen unterbringen, je nach Größe bzw. Auslegung der Lagerverpackung.

Wesentlich für die Lagerverpackung ist jedenfalls, dass diese nicht nur flüssigkeitsdicht sondern auch gasdicht, d. h. gasundurchlässig, ausgebildet ist. Dazu ist es von ganz besonderem Vorteil, wenn die Lagerverpackung aus aluminiumkaschiertem Kunststoff besteht, um nämlich die erforderliche Gasdichtigkeit gewährleisten zu können.

In vorteilhafter Weise ist zwischen dem Katheterschlauch und dem Gel ein Haftvermittler ausgebildet, der die Adhäsion des Gels an der Oberfläche des Katheters, zumindest im Verlaufe der Herstellung begünstigt. Auch ist es denkbar, dass zwischen der Oberfläche des Katheterschlauchs und dem Gel eine über die bloße Adhäsion hinausgehende Haftung durch Brückenbildung oder dgl. erzeugt wird. Auch chemische Reaktionen sind denkbar.

In weiter vorteilhafter Weise kann es sich bei dem die Oberflächenbeschichtung definierenden Gel um ein wasserbasiertes Gel handeln, welches sich einfach handhaben lässt.

Als Gel-Bildner lassen sich wasserlösliche Polymere verwenden, beispielsweise Cellulosederivate wie Hydroxyethylcellulose, Carboxymethylcellulose-Natrium, Hydroxypropylmethylcellulose oder Polyethylenoxide, Xanthan und/oder Polyvinylpyrrolidon. Andere geeignete Bildner lassen sich entsprechend dem Bedarf einsetzen.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Patentansprüche und andererseits auf die nachfolgende Erläuterung eines bevorzugten Ausführungsbeispiels der Erfindung anhand der Zeichnung zu verweisen. In Verbindung mit der Erläuterung des bevorzugten Ausführungsbeispiels der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel einer Gebrauchsverpackung mit darin befindlichem Gel-Katheter,
- Fig. 2: in einer schematischen Ansicht ein erfindungsgemäßes Katheter-Set, umfassend eine Lagerpackung mit drei darin befindlichen Gebrauchsverpackungen, die jeweils einen Gel-Katheter beinhalten und
- Fig. 3: in einer schematischen Ansicht eine geöffnete Gebrauchsverpackung mit herausgezogenem Gel-Katheter, wobei die sich an den Anschlussbereich des Katheters anschließende Gebrauchsverpackung zur schlauchartigen Verlängerung des Katheters dient.

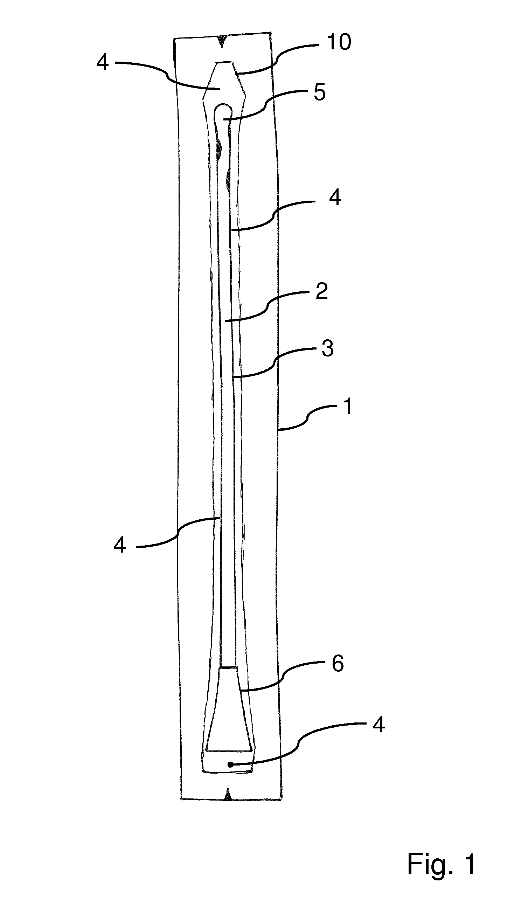

Fig. 1 zeigt in einer schematischen Ansicht einen in einer Gebrauchsverpackung 1 befindlichen Katheter 2, wobei es sich bei dem Katheter 2 um einen Katheter mit gelbeschichteter Oberfläche 3 handelt.

Die Oberfläche 3 des Katheters 2 weist eine Beschichtung mit einem mehr oder weniger dünnflüssigen Gel ähnlich einem Öl oder eines dünnflüssigen Fetts auf, wobei man aufgrund der dünnflüssigen Beschichtung bzw. Benetzung von einem ge wässerten Gel-Katheter sprechen kann.

Fig. 1 lässt des Weiteren erkennen, dass der Katheter 2 mit einer besonderen Katheterspitze 5 ausgestattet ist. Am gegenüberliegenden Ende des Katheters 2 ist ein Anschlussstück 6 vorgesehen.

Zur Gebrauchsverpackung 1 sei angemerkt, dass diese flüssigkeitsdicht, jedoch gasdurchlässig ausgeführt ist. Entsprechend ist es möglich, diese äußerst dünn, beispielsweise aus einer laminierten Folie, herzustellen, um eine möglichst hohe Flexibilität der Gebrauchsverpackung gewährleisten zu können.

Fig. 2 zeigt im Rahmen eines Ausführungsbeispiels eines erfindungsgemäßen Katheter-Sets eine Lagerpackung 7, die - wie in Fig. 2 angedeutet - drei Gebrauchsverpackungen 1 mit jeweils darin befindlichem Katheter 2 umfasst. Die in der Lagerverpackung 7 befindlichen Gebrauchsverpackungen 1 nebst Katheter sind lediglich angedeutet.

Die Lagerverpackung 4 ist aus aluminiumkaschiertem Kunststoff hergestellt und hat die Form eines Briefes bzw. einer flachen Tasche, so dass die darin befindlichen Gebrauchsverpackungen 1 mehr oder weniger nebeneinander angeordnet sind.

Die Lagerverpackung 7 ist gasundurchlässig ausgeführt, so dass aufgrund der Lagerverpackung 7 eine Langzeitlagerung der Katheter 2 möglich ist, ohne dass sich das Gel 4 verflüchtigt und somit an der Oberfläche des Katheters 2 austrocknet.

Fig. 2 zeigt des Weiteren, dass die Lagerverpackung 7 nach dem Aufreißen entlang der oberen Kante 8 wiederverschließbar ist, nämlich durch eine integrierte Druckleiste 9, die - ähnlich eines Reißverschlusses - ineinandergreifende Elemente umfasst. Durch Zusammendrücken lässt sich die Druckleiste 9 schließen.

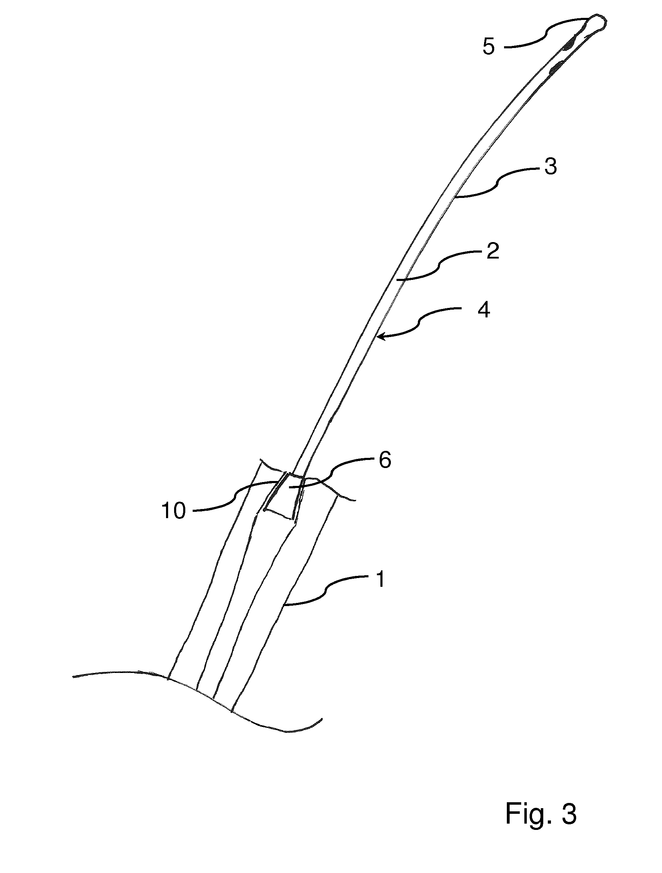

Fig. 3 zeigt den Katheter 2 im aus der Gebrauchsverpackung 1 herausgezogenen Zustand, wobei das konisch ausgestaltete Anschlussstück 6 in einem entsprechend konisch ausgestatteten Anschlussbereich 10 der Gebrauchsverpackung 1 klemmt. Somit stellt die schlauchartige Gebrauchsverpackung 1 eine Verlängerung des Katheters 2 dar und kann zur Urinableitung in eine Toilette verwendet werden.

In Bezug auf Merkmale, die sich den Fig. nicht entnehmen lassen, sei zur Vermeidung von Wiederholungen auf den allgemeinen Teil der Beschreibung verwiesen.

Schließlich sei angemerkt, dass das voranstehend erörterte Ausführungsbeispiel lediglich zur beispielhaften Erörterung der beanspruchten Lehre dient, diese jedoch nicht auf das Ausführungsbeispiel einschränkt.

### Bezugszeichenliste

- 1: Gebrauchsverpackung
- 2: Katheter, Katheterschlauch
- 3: gelbeschichtete Oberfläche (des Katheters)
- 4: Gel
- 5: Katheterspitze
- 6: Anschlusstück (des Katheters)
- 7: Lagerverpackung
- 8: obere Kante (der Lagerverpackung)
- 9: Druckleiste (der Lagerverpackung)
- 10: Anschlussbereich (der Gebrauchsverpackung)

## Patentansprüche

1. Katheter-Set umfassend einen zur Selbstkatheterisierung geeigneten Katheter (2) mit einer gelbeschichteten Oberfläche (3), wobei sich der Katheter (2) gebrauchsfertig in einer flüssigkeitsdichten, schlauchartigen Gebrauchsverpackung (1) befindet,
**dadurch gekennzeichnet, dass** die Gebrauchsverpackung (1) gasdurchlässig ausgebildet ist und dass mindestens zwei Gebrauchsverpackungen (1) mit jeweils darin befindlichem Katheter (2) gemeinsam in einer gasundurchlässigen Lagerverpackung (7) untergebracht sind.

2. Katheter-Set nach Anspruch 1, **dadurch gekennzeichnet, dass** sich der Katheter (2) in einem sterilen Zustand in der Gebrauchsverpackung (1) befindet.

3. Katheter-Set nach Anspruch 2, **dadurch gekennzeichnet, dass** der Katheter (2) vor oder nach seiner Anordnung in der Gebrauchsverpackung (1), nach einer herkömmlichen Methode, sterilisiert worden ist.

4. Katheter-Set nach Anspruch 2 oder 3, **dadurch gekennzeichnet, dass** der Katheter (2) vor oder nach der Kontaktierung mit dem Gel (4), nach einer herkömmlichen Methode, sterilisiert worden ist.

5. Katheter-Set nach einem der Ansprüche 1 bis 4, **dadurch gekennzeichnet, dass** die Gebrauchsverpackung (1) im Bereich der Katheterspitze (5) oder
in einem der Katheterspitze (5) abgewandten Bereich öffenbar bzw. aufreißbar ist, wobei
die Gebrauchsverpackung (1) derart ausgestaltet sein kann, dass der Katheter (2) mit der Katheterspitze (5) voran zumindest teilweise aus der geöffneten bzw. aufgerissenen Gebrauchsverpackung (1) herausschiebbar oder herausziehbar ist, wobei
der Katheter (2) mit einem endseitigen Anschlussstück (6) ausgestattet und im Wesentlichen bis zum Anschlussstück (6) aus der Gebrauchsverpackung (1) herausziehbar und mit dem Anschlussstück (6) dort klemmbar sein kann, wobei
sich das Anschlussstück (6) zum freien Ende hin konisch erweitern und dass die Gebrauchsverpackung (1) im öffenbaren Bereich eine entsprechend angepasste Innenwandung aufweisen kann, so dass sich das Anschlussstück (6) beim Herausschieben des Katheters (2) in diesen Bereich schieben und dort abdichtend festklemmen lässt und/oder wobei
das Anschlussstück (6) im herausgezogenen Zustand des Katheters (2) zur zumindest weitgehend abdichtenden Anlage an bzw. in der Gebrauchsverpackung (1) gelangt, dergestalt, dass die Gebrauchsverpackung (1) als schlauchartige Verlängerung des Katheters (2) zur unmittelbaren Urinableitung, beispielsweise in eine Toilette, dient.

6. Katheter-Set nach einem der Ansprüche 1 bis 5, **dadurch gekennzeichnet, dass** die Gebrauchsverpackung (1) aus einer laminierten Folie, vorzugsweise zweilagig, hergestellt ist, wobei
die Außenschicht der Folie aus Polyamid (PA) und die Innenschicht der Folie aus Polyethylen (PE) bestehen kann.

7. Katheter-Set nach einem der Ansprüche 1 bis 12, **dadurch gekennzeichnet, dass** die Folie dünn und flexibel ausgeführt ist, so dass sie sich zur Freigabe des Katheters (2) mühelos zusammenschieben bzw. zusammenraffen lässt.

8. Katheter-Set nach einem der Ansprüche 1 bis 7, **dadurch gekennzeichnet, dass** die Lagerverpackung (7) in Form eines lang gestreckten Briefs, Beutels oder dgl. ausgeführt ist.

9. Katheter-Set nach einem der Ansprüche 1 bis 14, **dadurch gekennzeichnet, dass** die Lagerverpackung (7) zum erstmaligen Öffnen an vorgegebener Stelle, vorzugsweise entlang einer Kante (8), aufreißbar ist und/oder
dass die Lagerverpackung (7) vorzugsweise über eine Druckleiste (9) oder dgl. reversibel öffenbar/schließbar ist.

10. Katheter-Set nach einem der Ansprüche 1 bis 9, **dadurch gekennzeichnet, dass** die Lagerverpackung (7) derart dimensioniert ist, dass sie zur Aufnahme von 5 bis 20 Gebrauchsverpackungen (1) mit jeweils einem Katheter (2) geeignet ist.

11. Katheter-Set nach einem der Ansprüche 1 bis 10, **dadurch gekennzeichnet, dass** die Lagerverpackung (7) aus aluminiumkaschiertem Kunststoff besteht.

12. Katheter nach einem der Ansprüche 1 bis 11, **dadurch gekennzeichnet, dass** zwischen dem Katheterschlauch (1) und dem Gel (4) ein Haftvermittler ausgebildet ist, zumindest aber wirkt.

13. Katheter nach einem der Ansprüche 1 bis 12, **dadurch gekennzeichnet, dass** es sich bei dem Gel (4) um ein wasserbasiertes Gel (4) handelt.

14. Katheter nach einem der Ansprüche 1 bis 13, **dadurch gekennzeichnet, dass** als Gel-Bildner wasserlösliche Polymere, beispielsweise Cellulosederivate wie Hydroxyethylcellulose, Carboxymethylcellulose-Natrium, Hydroxypropylmethylcellulose oder Polyethylenoxide, Xanthan und/oder Polyvinylpyrrolidon verwendet werden.
